(19) Europäisches Patentamt
European Patent Office
Office européen des brevets

(11) **EP 2 848 193 B1**

(12) **EUROPEAN PATENT SPECIFICATION**

(45) Date of publication and mention
of the grant of the patent:
**07.12.2022 Bulletin 2022/49**

(21) Application number: **14183820.1**

(22) Date of filing: **05.09.2014**

(51) International Patent Classification (IPC):
**A61B 5/021** *(2006.01)* **G06T 7/20** *(2017.01)*
**A61B 5/00** *(2006.01)* **G06T 7/00** *(2017.01)*

(52) Cooperative Patent Classification (CPC):
**A61B 5/02125; A61B 5/7239; A61B 5/7278;**
**G06T 7/20;** A61B 5/0077; A61B 2576/00;
G06T 2207/10016; G06T 2207/10024;
G06T 2207/30076; G16H 30/40

(54) **SYSTEM AND METHOD FOR DETERMINING VIDEO-BASED PULSE TRANSIT TIME WITH TIME-SERIES SIGNALS**

SYSTEM UND VERFAHREN ZUR BESTIMMUNG EINER VIDEOBASIERTEN PULSTRANSITZEIT AUS ZEITREIHEN SIGNALEN

SYSTÈME ET PROCÉDÉ DE DÉTERMINATION VIDÉO DU TEMPS DE TRANSIT DU POULS À L'AIDE DE SIGNAUX DE SÉRIES TEMPORELLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.09.2013 US 201314026739**

(43) Date of publication of application:
**18.03.2015 Bulletin 2015/12**

(73) Proprietor: **Xerox Corporation
Norwalk, CT 06856-4505 (US)**

(72) Inventors:
• **Mestha, Lalit Keshav
  Fairport, New York 14450 (US)**
• **Kyal, Survi
  Rochester, New York 14623 (US)**

(74) Representative: **Gill Jennings & Every LLP
The Broadgate Tower
20 Primrose Street
London EC2A 2ES (GB)**

(56) References cited:
**US-A1- 2013 218 028**

• **Gary F Mitchell ET AL: "J Appl Physiol", Journal of Applied Physiology, 1 January 1997 (1997-01-01), pages 203-210, XP055135302, Retrieved from the Internet: URL:http://jap.physiology.org/content/jap/82/1/203.full.pdf [retrieved on 2014-08-19]**
• **ASHIS MOOKERJEE ET AL: "Arterial pulse wave velocity measurement: different techniques, similar results-implications for medical devices", BIOMECHANICS AND MODELING IN MECHANOBIOLOGY, SPRINGER, BERLIN, DE, vol. 9, no. 6, 7 April 2010 (2010-04-07), pages 773-781, XP019859875, ISSN: 1617-7940, DOI: 10.1007/S10237-010-0213-Y**
• **F Lopes Da Silva ET AL: "Interdependence of EEG signals: linear vs. nonlinear associations and the significance of time delays and phase shifts", Brain topography, 30 September 1989 (1989-09-30), pages 9-18, XP055157190, UNITED STATES DOI: 10.1007/BF01128839 Retrieved from the Internet: URL:http://www.ncbi.nlm.nih.gov/pubmed/2641479 [retrieved on 2014-12-08]**

**EP 2 848 193 B1**

## Description

## Technical Field

The present invention is directed to systems and methods for determining the patient's arterial pulse transit time from a source video signal acquired of that patient.

## Background

The ability to capture physiological signals by non-contact means is highly desirable in the healthcare industry. One physiological signal of importance is the pulse transit time for many reasons, one of which is that the pulse transit time has a correlation with blood pressure. To obtain such measurements, electrodes of an electro-cardiogram (ECG) device need to be attached directly to the patient's skin. This can be a problem in neonatal intensive care units caring for premature babies with sensitive skin.

Accordingly, what is needed in this art are systems and methods for determining a subject of interest's arterial pulse transit time from time-varying source signals generated from video images.

US 2013/0218028 A1 discloses a system and method for determining an arterial pulse transit time of a subject of interest in a remote sensing environment. A video imaging system is used to capture a time varying source images of a proximal and distal region of a subject intended to be analyzed for arterial pulse transit time. A time series signal for each of the proximal and distal regions is extracted from the source images and a phase of each of the extracted time series signals is computed. A difference is then computed between these phases. This phase difference is a monotonic function of frequencies in the signals. From the monotonic function, an arterial pulse transit time of the subject is extracted.

The following U.S. Patents, U.S. Patent Applications, and Publications are also regarded as relevant prior art.

*"Estimating Cardiac Pulse Recovery From Multi-Channel Source Data Via Constrained Source Separation"*, U.S. Patent Application Serial No. 13/247,683, by Mestha et al.

*"Filtering Source Video Data Via Independent Component Selection"*, U.S. Patent Application Serial No. 13/281,975, by Mestha et al.

*"Method For Classifying A Pixel Of A Hyperspectral Image In A Remote Sensing Application"*, U.S. Patent Application Serial No. 13/023,310, by Mestha et al.

*"Determining A Total Number Of People In An IR Image Obtained Via An IR Imaging System"*, U.S. Patent Application Serial No. 12/967,775, by Wang et al, which discloses a ratio method for classifying pixels in an IR image.

*"Determining A Number Of Objects In An IR Image"*, U.S. Patent Application Serial No. 13/086,006, by Wang et al, which discloses a correlation method and a best fitting reflectance method for classifying pixels in an IR image.

*"Post-Processing A Multi-Spectral Image For Enhanced Object Identification"*, U.S. Patent Application Serial No. 13/324,368, by Wang et al.

*"Removing Environmental Factors From Signals Generated From Video Images Captured For Biomedical Measurements"*, U.S. Patent Application Serial No. 13/401,207, by Mestha et al.

## Brief Summary

What is disclosed is a system and method for determining a subject of interest's arterial pulse transit time from time-varying source signals generated from video images. In one embodiment, a video imaging system is used to capture a time-varying source signal of a proximal and distal region of a subject of interest. The image frames are processed to isolate localized areas of a proximal and distal region of exposed skin of the subject. A time-series signal for each of the proximal and distal regions is extracted from the source video. A phase angle is computed with respect to frequency for each of the time-series signals to produce respective phase v/s frequency curves for each region. Slopes within a selected cardiac frequency range are then extracted from each of the phase v/s frequency curves. A difference is computed between the two slopes to obtain an arterial pulse transit time for the subject. Accordingly, there is provided a method according to claim 1 and a system according to claim 10.

The video imaging system of claim 10 may be used to acquire said time-varying source images of said proximal and distal regions comprises two video cameras, a first video camera acquiring a first video of said proximal region and a second video camera acquiring a second video of said distal region.

Many features and advantages of the above-described method will become readily apparent from the following detailed description and accompanying drawings.

## Brief Description of the Drawings

The foregoing and other features and advantages of the subject matter disclosed herein will be made apparent from the following detailed description taken in conjunction with the accompanying drawings, in which:

FIG. 1 shows a subject of interest's right arm extremity clutching a pole to illustrate two proximal and distal points in the arterial system of the human arm;
FIG. 2 is a flow diagram which illustrates one embodiment of the present method for determining an arterial pulse transit time between a proximal and distal region of a subject of interest from source video images acquired using a video imaging system;

FIG. 3 is an image of a subject with a proximal region identified as being a region of the back of the hand and a distal region identified as being a region of the face;

FIG. 4 shows the plotted slopes for the phase angle with respect to frequency curves obtained from having processed the time-varying source signals for each of the proximal and distal regions of FIG. 3, in accordance with the teachings hereof; and

FIG. 5 is a block diagram of an example networked image processing system wherein various aspects of the present method, as described in the flow diagram of FIG. 2, are implemented.

## Detailed Description

What is disclosed is a system and method for determining a subject of interest's arterial pulse transit time from time-varying source signals generated from video images.

## NON-LIMITING DEFINITIONS

A *"subject of interest'*, as used herein, refers to any subject which is capable of registering an arterial pulse. The present method applies equally to any subjects capable of registering an arterial pulse. Use of terms herein for explanatory purpose, such as "person" or "patient", are not to be viewed as limiting the scope of the appended claims solely to human subjects.

*"Proximal"* (from the Latin *proximus:* meaning nearest to) refers to a point that is nearer to the source of the arterial pulse which is in the ascending aorta. Note that the systemic arterial system originate from the aorta. As left ventricle of the heart contracts blood exits from the ascending aorta in the form of waves and flows into systemic arteries. The heart is located near the anterior chest wall, directly posterior to the sternum. For arterial pulse measurement, a proximal point in an artery is a point which is closer to the heart, i.e., upstream from the distal point.

*"Distal"* (from the Latin *distare:* meaning away from) refers to a point that is farther from a center of the body. For arterial pulse measurement purposes, the distal point in the artery is a point which is farther from the heart, i.e., upstream or downstream from the proximal point as the arterial network carries blood upstream & downstream through the branches of the aortic arch and the descending aorta. By drawing an imaginary line between the proximal and distal points, a proximo-distal axis is created. The elbow is proximal to the wrist but distal to the shoulder since the blood flows from brachial arteries towards the wrist through radial and ulnar arteries. Blood vessels may also be labeled as *"ostial'* (referring to point where the blood vessel branches off) and "distal" (referring to a point away from the branch point).

An *"arterial pulse wave"* is a pressure wave created throughout the vascular system when the left ventricle of the heart muscle contracts and pushes a volume of blood into the aorta. This generates a perturbation that travels from the heart and down into the arterial network. An arterial pulse wave has two primary components, i.e., a forward traveling wave when the left ventricle contracts, and a reflected wave returning back from the peripheral. The actual pressure in the aorta is the sum of the initial wave and the reflected wave.

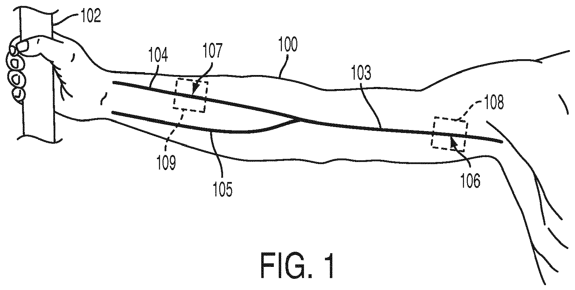

The *"pulse transit time"* refers to the time it takes a pressure pulse to travel from a proximal arterial site to a distal arterial site. Pulse transit time (PTT) is a function of the pulse wave velocity which, in turn, is a function of the blood pressure, vessel diameter, blood density. Localized PTT is used as an indirect marker of various pathologic conditions. PTT has high correlations when utilized as a surrogate monitor of BP changes. Present literature suggests that description of disease-specific cardiovascular reactivity pattern is feasible with techniques that are based upon PTT. PTT signals can be calibrated to extract beat-to-beat blood pressure and blood velocity in the patient's vascular network including facilitating for variety of diagnosis such as, for example, blood vessel dilation over time, vessel blockage between two points (or regions) of interest, peripheral neuropathy for diabetic patients etc. FIG. 1 shows a subject of interest's right arm 100 extended outward and clutching a section of a pole 102. The subject's brachial artery 103 extends down the arm and branches into the radial and ulnar arteries, at 104 and 105 respectively. A point 106 in the brachial artery is proximal to a point 107 in the radial artery. In FIG. 1 and for discussion purposes, the pulse transit time is the time it takes for the arterial pulse wave to travel from point 106 in proximal region 108 to point 107 in distal region 109. When face and hand regions are selected, since there is no vascular network directly connecting between face and the hand, the video-based pulse transit time takes the meaning of time difference between face signal and hand signal.

*"Source video images"* are a time-varying sequence of images acquired using a video imaging sensor. A source video image can be any combination of: NIR images, RGB images, RGB with NIR images, thermal, multi-spectral images, and hyperspectral video images. It should be appreciated that when the video capture is made in the NIR band, enough illumination will be required to image in the infrared wavelength.

A *"video imaging sensor"* refers to a device for acquiring source video data over one or more channels. The video imaging sensor may be a device with a high frame rate and high spatial resolution such as, for example, a monochrome camera for capturing black/white video images, or a color camera for capturing color video images. The video imaging sensor may be a spectral sensor such as a thermal, multi-spectral or hyperspectral system. The imaging sensor may be a hybrid device capable of operating in a conventional video mode with high frame rates and high spatial resolution, and a spectral mode with low frame rates but high spectral resolution. Video imaging sensors comprising standard video cameras and those comprising specialized sensors are readily available from a wide array of vendors in various streams of commerce.

A *"remote sensing environment'* refers to non-contact, non-invasive sensing, i.e., the imaging sensor does not physically contact the subject being sensed. The environment may be any settings such as, for example, a hospital, ambulance, medical office, and the like.

**Example Flow Diagram of One Embodiment**

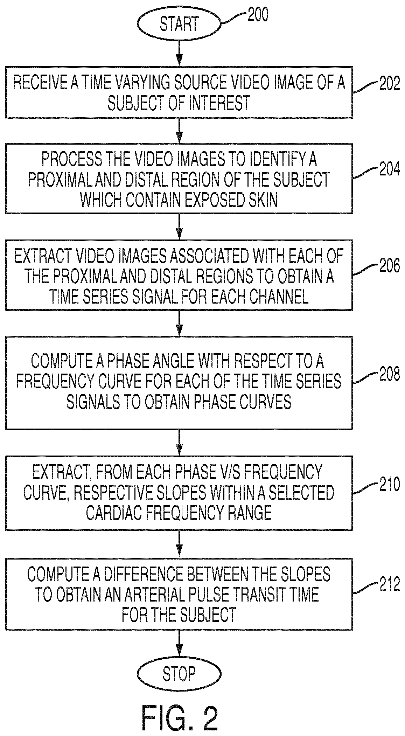

Reference is now being made to the flow diagram of FIG. 2 which illustrates one embodiment of the present method for determining an arterial pulse transit time between a proximal and distal region of a subject of interest from source video images acquired using a video imaging system. Flow processing begins at step 200 and immediately proceeds to step 202.

At step 202, a time-varying source images is received which has been captured of a subject of interest. The source video images have been acquired over at least one channel of a video imaging system. The source images comprise a plurality of image frames.

At step 204, the video images are processed to identify a proximal and a distal region of the subject which contain areas of exposed skin. Example proximal and distal regions are shown and described with respect to regions 108 and 109 of FIG. 1, respectively. Areas of exposed skin can be determined in an image by analyzing the video images, on a frame-by-frame basis, and classifying the pixels comprising in those image frames. Pixels classified as human skin can be identified using, for example, the above-incorporated pixel classification methods.

At step 206, video images associated with each of the identified proximal and distal regions are extracted from the source images to obtain a time-series signal for each channel. The signal corresponds to volumetric changes in blood pressure. This can be effectuated by computing an average of all pixels in each of the identified proximal and distal regions within each image frame to obtain a channel average per frame for each of the regions. A global channel average can then be computed, for each channel, by adding the channel averages across multiple frames and dividing by the total number of frames. The channel average is subtracted from the global channel average and the result is divided by a global channel standard deviation to obtain a zero-mean unit variance time-series signal for each of the proximal and distal regions. These time-series signals contain frequency components. For the purpose of determining the pulse transit time, processing with only a single color channel is adequate. For example, in a RGB video, time-series signals from the green channel contain a sufficient signal. Once normalized time-series signals have been obtained for each of the proximal and distal regions, these are then subjected to a pre-filtering to remove undesirable frequencies using a Fast Fourier Transform (FFT) algorithm. The resulting pre-processed and pre-filtered time-series signals contain the sum total of volumetric pressure changes within each region. Further pre-processing can be done to extract the source blood volume signals (such as the plethysmographic signals) using blind source separation algorithms such as the independent component analysis or the constrained independent component analysis as described in the aforementioned references. It should be understood that the volume changes in the first and second regions are due to all the blood vessels in each of those regions. Arterial pulsations are a dominant component in these signals. Components from smaller structures such as capillaries and terminal arterioles although are less significant as these only provide a minor contribution to the registered pulsations, but may be significant when the region of interest is large as the integrated effect may improve signal to noise ratio. If camera related noise or other environmental factors affecting the video capture are present, compensation can be introduced as described in the above mentioned reference entitled: *"Removing Environment Factors From Signals Generated From Video Images Captured For Biomedical Measurements"*, By Mestha et al. The post-compensated signal contains decorrelated and noise corrected channels, i.e., environmentally compensated signals for each of the proximal and distal regions.

At step 208, compute a phase angle with respect to frequency for each of the time-series signals to obtain respective phase v/s frequency curves of frequencies within these signals. Only one of the video channels can be used to determine the phase v/s frequency curves. If a source separation algorithm is used then the source signals from proximal and distal regions will be used to compute the phase v/s frequency curves. One of ordinary skill will readily understand how to compute a phase angle $\varphi(\omega)$ with respect to frequency $\omega$ given a time-series signal. If $\varphi_1(\omega)$ is the phase of the time-series signal corresponding to the proximal region and $\varphi_2(\omega)$ is the phase of the time-series signal corresponding to the distal region then $T_1 = \partial\varphi_1/\partial\omega$ and $T_2 = \partial\varphi_2/\partial\omega$ are respective slopes. These slopes may be computed by fitting a polynomial equation to each of the phase v/s frequency curves or by taking derivative with respect to frequency. If the phase with respect to frequency curve is linear then all the frequencies contained within the time-series signal have same time shift.

At step 210, extract, from each phase v/s frequency curves, respective slopes within a selected cardiac frequency range. One cardiac frequency range is 0.75 to 4.0 Hz. Slopes represent time shift of each harmonics within the respective time-series signal.

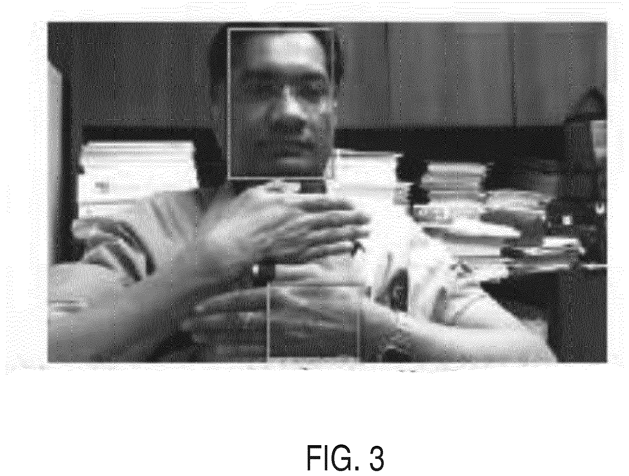

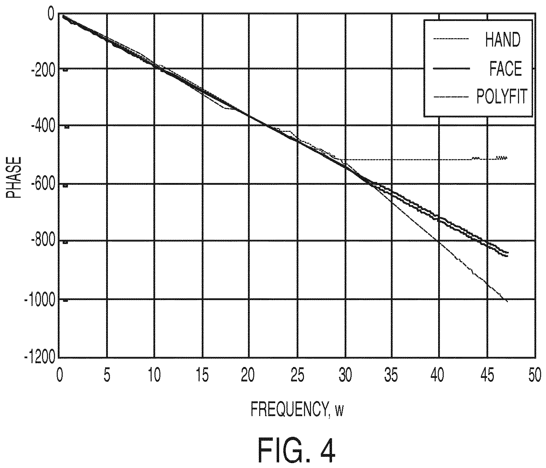

At step 212, compute a difference between the slopes to obtain an arterial pulse transit time for the subject. In this embodiment, further processing stops. FIG. 3 shows an image of a subject with a distal region identified as being a region of the back of the hand and a proximal region identified as being a region of the subject's face. FIG. 4 shows the plotted phase v/s frequency curves with respect to frequency obtained for each of the proximal and distal regions of FIG. 3. These phase v/s frequency curves exhibit linear characteristics with respect to frequency within the cardiac frequency range. The slope of the hand region is -18.0917 seconds. The slope of the facial region is -17.5271 seconds. The computed difference corresponding to the subject's arterial pulse transit time between the proximal and distal regions is 0.5647 sec. The slope difference of the two linear phase v/s frequency curves corresponds to the propagation (or time) delay for the blood to flow between these two regions. It is to be noted that the phase v/s frequency curves may not be linear in all subjects of varying levels of disease state.

In other embodiments, the arterial pulse transit time is communicated to a computer system and used to facilitate a determination of any of: a blood pressure in the subject's vascular network, a blood vessel dilation over time, a blood vessel blockage, a blood flow velocity, and/or the existence of a peripheral neuropathy.

It should be appreciated that the flow diagrams hereof are illustrative. One or more of the operative steps illustrated in the flow diagram may be performed in a differing order. Other operations, for example, may be added, modified, enhanced, condensed, integrated, or consolidated. Such variations are intended to fall within the scope of the appended claims. All or portions of the flow diagrams may be implemented partially or fully in hardware in conjunction with machine executable instructions.

**Source Signal Processing System**

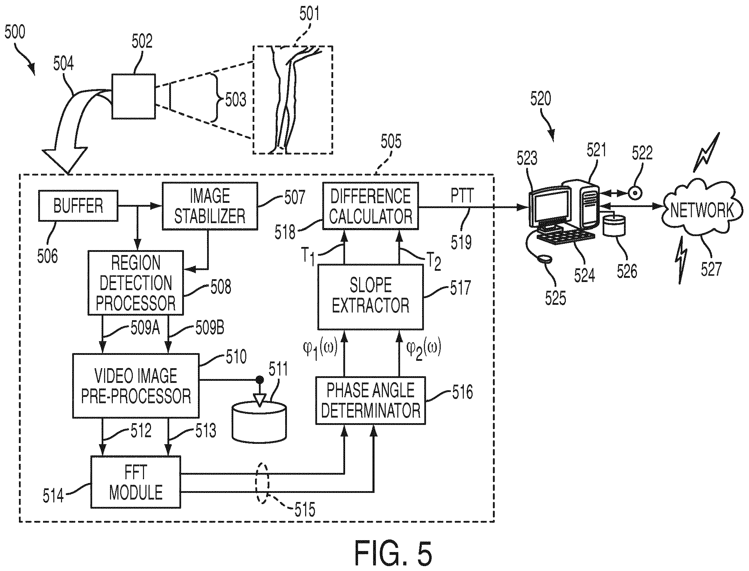

Reference is now being made to FIG. 5 which is a block diagram of an example networked video image processing system 500 wherein various aspects of the present method as described with respect to the flow diagram of FIG. 2 are implemented.

In FIG. 5, imaging sensor 502 acquires source video images 501 of a subject of interest (shown as a right arm) captured in the video camera's field of view 503. The source video images are acquired over at least one imaging channel and the signals 504 for each channel are communicated to Video Image Processing System 505 wherein various aspects of the present method are performed.

System 505 is shown comprising a Buffer 506 for buffering the source signal for processing. Buffer 506 may further store data, formulas, mathematical representations, and the like, as are needed to process the source video images in accordance with the teachings hereof. Image Stabilizer Module 507 processes the images to compensate, where needed, for anomalies such as motion induced blur, imaging blur, slow illuminant variation, and the like. Region Detection Processor 508 receives the video images and processes the images contained therein in order to determine a proximal and a distal region, at 509A and 509B, respectively. One or more frames of the captured source video image may be communicated by pathways not shown to workstation 520 for display 523 thereon such that the user can select any of the proximal and distal regions from any of the captured image frames. A selection may be made by the user using, for example, a rubber-band box created by clicking and dragging a mouse or by otherwise highlighting localized areas of exposed skin in one or more image frames of the video sequence for processing.

Portions of the source images associated with each of the proximal and distal regions are provided to Video Image Pre-Processor 510 which receives the source images associated with each of the identified proximal and distal regions and extracts a time-series signal from the source images for each channel of each region. Various signal components may be stored/retrieved to storage device 511 using communication pathways not shown. The normalized time-series signals 512 and 513, extracted for the proximal and distal regions respectively, are provided to FFT Module 514 wherein these time-series signals are subjected to a pre-filtering to remove undesirable frequencies. FFT Module may also use other filtering techniques. The filtered time-series signals, collectively at 515, are provided to Phase Angle Determinator 516 which receives the filtered time-series signals for each of the proximal and distal regions and computes a phase $\varphi$ with respect to frequency for each of the time-series signals for a selected channel of interest to obtain phase v/s frequency curves. Slope Extractor 517 receives the phase v/s frequency curves for the proximal and distal regions, $\varphi_1(\omega)$ and $\varphi_2(\omega)$, respectively, and extracts, from each of the phase v/s frequency curves, slopes ($T_1$ and $T_2$) within a selected cardiac frequency range. Difference Calculator 518 computes a difference between the two slopes to obtain an arterial pulse transit time (PTT) 519 for the subject. The arterial pulse transit time is communicated to networked computer system 520.

Workstation 520 reads/writes to computer readable media 522 such as a floppy disk, optical disk, CD-ROM, DVD, magnetic tape, etc. Case 521 houses a motherboard with a processor and memory, a network card, graphics card, and the like, and other software and hardware. The workstation includes a user interface which, in this embodiment, comprises display 523 such as a CRT, LCD, touch screen, etc., a keyboard 524 and a mouse 525. A user or technician may use the keyboard and/or mouse to identify the proximal and distal regions, set parameters, select images for processing, view results, and the like. It should be appreciated that the workstation has an operating system and other specialized software configured to display a variety of numeric values, text, scroll bars, pull-down menus with user selectable options, and the like, for entering, selecting, or modifying information displayed on display device 523. Various portions of the source video signals captured by video capture device 502 may be communicated to workstation 520 for processing and stored to storage device 526. Workstation 520 is in communication with one or more remote devices of network 527 via a communications interface internal to case 521.

It should be appreciated that some or all of the functionality performed by any of the modules and processing units of the signal processing system 505 can be performed, in whole or in part, by workstation 520. Any of these may be stored to storage device 526 or written to computer media 522. Any of the modules and processing units of FIG. 5 can be placed in communication with storage devices 511 and 526 and may store/retrieve therefrom data, variables, records, parameters, functions, machine readable/executable program instructions required to perform their intended functions. Each of the modules of system 505 may be placed in communication with one or more devices over network 527. Although shown as a desktop computer, it should be appreciated that computer system 520 can be any of a laptop, mainframe, server, or a special purpose computer such as an ASIC, circuit board, dedicated processor, or the like.

It should also be appreciated that various modules may designate one or more components which may, in turn, comprise software and/or hardware designed to perform the intended function. A plurality of modules may collectively perform a single function. Each module may have a specialized processor capable of executing machine readable program instructions. A module may comprise a single piece of hardware such as an ASIC, electronic circuit, or special purpose processor. A plurality of modules may be executed by either a single special purpose computer system or a plurality of special purpose computer systems operating in parallel. Connections between modules include both physical and logical connections. Modules may further include one or more software/hardware modules which may further comprise an operating system, drivers, device controllers, and other apparatuses some or all of which may be connected via a network. It is also contemplated that one or more aspects of the present method may be implemented on a dedicated computer system and may also be practiced in distributed computing environments where tasks are performed by remote devices that are linked through a network. The teachings hereof can be implemented in hardware or software using any known or later developed systems, structures, devices, and/or software by those skilled in the applicable art without undue experimentation from the functional description provided herein with a general knowledge of the relevant arts.

## Various Embodiments

One or more aspects of the methods described herein are intended to be incorporated in an article of manufacture, including one or more computer program products, having computer usable or machine readable media. The article of manufacture may be included on at least one storage media readable by a machine architecture or an image processing system embodying executable program instructions capable of performing the methodology described herein. The article of manufacture may be included as part of an operating system and may be shipped, sold, leased, or otherwise provided separately, either alone or as part of an add-on, update, upgrade, or product suite. The invention is defined by appended claims 1-14.

## Claims

1. A method, implemented by a processor, for determining an arterial pulse transit time between a proximal and distal region of a subject of interest from source video images acquired using a video imaging system, the method comprising:

   receiving time-varying source images acquired over at least one channel of a video imaging system, said source images comprising video images captured of a proximal and distal region of an area of exposed skin of a subject of interest, each of the proximal and distal regions being large enough such that the contribution of capillaries and terminal arterioles improves the signal to noise ratio, wherein the proximal region is the back of a hand of the subject, and the distal region is the face of the subject;
   extracting, from said source images, a first time-series signal for said proximal region and a second times series signal for said distal region;
   computing a first phase angle $\varphi_1(\omega)$ with respect to frequency $\omega$ of said time-series signal for said proximal region to obtain a first phase vs frequency curve;
   computing a second phase angle $\varphi_2(\omega)$ with respect to frequency $\omega$ of said time-series signal for said distal region to obtain a second phase vs frequency curve;
   computing respective slopes $T_1 = \frac{\partial \varphi_1}{\partial \omega}$ and $T_2 = \frac{\partial \varphi_2}{\partial \omega}$ within a defined cardiac frequency range between 0.75 to 4.0 Hz from each of said respective phase vs frequency curves;
   computing a difference between said slopes $T_1$

and $T_2$, said difference corresponding to an arterial pulse transit time for said subject between said proximal and distal regions; and storing said arterial pulse transit time to a storage device.

2. The method of claim 1, wherein said time-varying source signal comprises any combination of: NIR images, RGB images, RGB with NIR images, multispectral images, thermal images, and hyperspectral images.

3. The method of claim 1, wherein said proximal and distal regions comprise, respectively, a first and second localized area of said subject.

4. The method of claim 1, further comprising determining, from said arterial pulse transit time, any of: a blood pressure in said subject's vascular network, a blood vessel dilation over time, a blood vessel blockage, a blood flow velocity, and the existence of a peripheral neuropathy.

5. The method of claim 1, wherein said video imaging system used to acquire said time-varying source images of said proximal and distal regions comprises two video cameras, a first video camera acquiring a first video of said proximal region and a second video camera acquiring a second video of said distal region.

6. The method of claim 1, wherein said time-varying source is a single color channel.

7. The method of claim 6, wherein said single color channel is a green color channel.

8. The method of claim 1, wherein said computing said respective slopes includes fitting a polynomial equation to each of said phase vs frequency curves.

9. The method of claim 1, wherein said computing said respective slopes includes computing a derivative with respect to a frequency.

10. A video based system for determining an arterial pulse transit time between a proximal and distal region of a subject of interest from source video images acquired using a video camera, the system comprising:

a video imaging system for capturing a time-varying source images over at least one channel, said source images comprising video images captured of a proximal and distal region of an exposed area of skin of a subject of interest, each of the proximal and distal regions being large enough such that the contribution of capillaries and terminal arterioles improves the signal to noise ratio wherein the proximal region is the back of a hand of the subject, and the distal region is the face of the subject; and a processor in communication with said video camera and a memory, said processor being configured to execute machine readable instructions for performing:

receiving a time-varying source images acquired over at least one channel of said video imaging system, said source images comprising video images captured of a proximal and distal region of an area of exposed skin of a subject of interest; extracting, from said source images, a first time-series signal for said proximal region and a second times series signal for said distal region; computing a first phase $\varphi_1(\omega)$ angle with respect to frequency $\omega$ of said time-series signal for said proximal region to obtain a first phase vs frequency curve; computing a second phase angle $\varphi_2(\omega)$ with respect to frequency $\omega$ of said time-series signal for said distal region to obtain a second phase vs frequency curve; computing respective slopes

$$T_1 = \frac{\partial \varphi_1}{\partial \omega} \text{ and } T_2 = \frac{\partial \varphi_2}{\partial \omega}$$

within a defined cardiac frequency range between 0.75 to 4.0 Hz from each of said respective phase vs frequency curves; computing a difference between said slopes $T_1$ and $T_2$, said difference corresponding to an arterial pulse transit time for said subject between said proximal and distal regions; and storing said arterial pulse transit time to said memory.

11. The system of claim 10, wherein said time-varying source signal comprises any combination of: NIR images, RGB images, RGB with NIR images, multispectral images, thermal images, and hyperspectral images.

12. The system of claim 10, wherein said proximal and distal regions comprise, respectively, a first and second localized area of said subject.

13. The system of claim 10, further comprising determining, from said arterial pulse transit time, any of: a blood pressure in said subject's vascular network, a blood vessel dilation over time, a blood vessel blockage, a blood flow velocity, and the existence of a peripheral neuropathy.

**14.** The system of claim 10, wherein said video imaging system used to acquire said time-varying source images of said proximal and distal regions comprises two video cameras, a first video camera for acquiring a first video of said proximal region and a second video camera for acquiring a second video of said distal region

**Patentansprüche**

**1.** Verfahren, das von einem Prozessor implementiert wird, zum Bestimmen einer arteriellen Pulswellenlaufzeit zwischen einem proximalen und einem distalen Bereich eines untersuchten Patienten anhand unter Einsatz eines Video-Bildgebungssystems erfasster Quellen-Videobilder, wobei das Verfahren umfasst:

Empfangen zeitlich variierender Quellen-Bilder, die über wenigstens einen Kanal eines Video-Bildgebungssystems erfasst werden, wobei die Quellen-Bilder Videobilder umfassen, die von einem proximalen und einem distalen Bereich einer Fläche freiliegender Haut eines untersuchten Patienten aufgenommen werden, wobei der proximale und der distale Bereich jeweils so groß sind, dass der Beitrag von Kapillaren sowie terminalen Arteriolen das Signal-Rausch-Verhältnis verbessert, und der proximale Bereich der Handrücken des Patienten ist und der distale Bereich das Gesicht des Patienten ist;
Extrahieren eines ersten Zeitreihen-Signals für den proximalen Bereich und eines zweiten Zeitreihen-Signals für den distalen Bereich aus den Quellen-Bildern;
Berechnen eines ersten Phasenwinkels $\varphi_1(\omega)$ in Bezug auf die Frequenz $\omega$ des Zeitreihen-Signals für den proximalen Bereich, um eine erste Phasen-Frequenz-Kurve zu ermitteln;
Berechnen eines zweiten Phasenwinkels $\varphi_2(\omega)$ in Bezug auf die Frequenz $\omega$ des Zeitreihen-Signals für den distalen Bereich, um eine zweite Phasen-Frequenz-Kurve zu ermitteln;
Berechnen jeweiliger Anstiege

$$T_1 = {}^{\delta\varphi1}/_{\delta\omega} \text{ bzw. } T_2 = {}^{\delta\varphi2}/_{\delta\omega}$$ innerhalb eines definierten Herzfrequenzbereiches zwischen 0,75 und 4,0 Hz anhand jeder der jeweiligen Phasen-Frequenz-Kurven;
Berechnen einer Differenz zwischen den Anstiegen $T_1$ und $T_2$, wobei die Differenz einer arteriellen Pulswellenlaufzeit für den Patienten zwischen dem proximalen und dem distalen Bereich entspricht; und
Speichern der arteriellen Pulswellenlaufzeit in einer Speichervorrichtung.

**2.** Verfahren nach Anspruch 1, wobei das zeitlich variierende Quellen-Signal eine beliebige Kombination aus NIR-Bildern, RGB-Bildern, RGB- mit NIR-Bildern, multispektralen Bildern, Wärmebildern und hyperspektralen Bildern umfasst.

**3.** Verfahren nach Anspruch 1, wobei der proximale und der distale Bereich jeweils eine erste bzw. eine zweite lokal begrenzte Fläche des Patienten umfassen.

**4.** Verfahren nach Anspruch 1, das des Weiteren umfasst, dass anhand der arteriellen Pulswellenlaufzeit ein Blutdruck im Gefäßnetz des Patienten, eine Erweiterung der Blutgefäße im Verlauf der Zeit, eine Blutgefäß-Verstopfung oder/und das Vorhandensein einer peripheren Neuropathie bestimmt wird/werden.

**5.** Verfahren nach Anspruch 1, wobei das Video-Bildgebungssystem, das zum Erfassen der zeitlich variierenden Quellen-Bilder des proximalen und des distalen Bereiches eingesetzt wird, zwei Videokameras, das heißt eine erste Videokamera, die ein erstes Videobild des proximalen Bereiches erfasst, und eine zweite Videokamera umfasst, die ein zweites Videobild des distalen Bereiches erfasst.

**6.** Verfahren nach Anspruch 1, wobei die zeitlich variierende Quelle ein einzelner Farbkanal ist.

**7.** Verfahren nach Anspruch 6, wobei der einzelne Farbkanal ein Grün-Farbkanal ist.

**8.** Verfahren nach Anspruch 1, wobei das Berechnen der jeweiligen Anstiege Angleichen einer Polynomgleichung an jede der Phasen-Frequenz-Kurven umfasst.

**9.** Verfahren nach Anspruch 1, wobei das Berechnen der jeweiligen Anstiege Berechnen einer Ableitung in Bezug auf eine Frequenz umfasst.

**10.** Videobasiertes System zum Bestimmen einer arteriellen Pulswellenlaufzeit zwischen einem proximalen und einem distalen Bereich eines untersuchten Patienten anhand unter Einsatz einer Videokamera erfasster Quellen-Videobilder, wobei das System umfasst:

ein Video-Bildgebungssystem zum Aufnehmen zeitlich variierender Quellen-Bilder über wenigstens einen Kanal, wobei die Quellen-Bilder Videobilder umfassen, die von einem proximalen und einem distalen Bereich einer Fläche freiliegender Haut eines untersuchten Patienten aufgenommen werden, wobei der proximale und der distale Bereich jeweils so groß sind, dass der Beitrag von Kapillaren sowie terminalen Arteriolen das Signal-Rausch-Verhältnis verbessert, und der proximale Bereich der Handrücken des Patienten ist und der distale Bereich das Gesicht des Patienten ist;
einen Prozessor, der mit der Videokamera und einem Speicher in Kommunikationsverbindung steht, wobei der Prozessor so konfiguriert ist, dass er maschinenlesbare Anweisungen ausführt, um durchzuführen:

Empfangen zeitlich variierender Quellen-Bilder, die über wenigstens einen Kanal des Video-Bildgebungssystems erfasst werden, wobei die Quellen-Bilder Videobilder umfassen, die von einem proximalen und einem distalen Bereich einer Fläche freiliegender Haut eines untersuchten Patienten aufgenommen werden;
Extrahieren eines ersten Zeitreihen-Signals für den proximalen Bereich und eines zweiten Zeitreihen-Signals für den distalen Bereich aus den Quellen-Bildern;
Berechnen eines ersten Phasenwinkels $\varphi_1(\omega)$ in Bezug auf die Frequenz $\omega$ des Zeitreihen-Signals für den proximalen Bereich, um eine erste Phasen-Frequenz-Kurve zu ermitteln;
Berechnen eines zweiten Phasenwinkels $\varphi_2(\omega)$ in Bezug auf die Frequenz $\omega$ des Zeitreihen-Signals für den distalen Bereich, um eine zweite Phasen-Frequenz-Kurve zu ermitteln;
Berechnen jeweiliger Anstiege $T_1 = {}^{\delta\varphi_1}\!/_{\delta\omega}$ bzw. $T_2 = {}^{\delta\varphi_2}\!/_{\delta\omega}$ innerhalb eines definierten Herzfrequenzbereiches zwischen 0,75 und 4,0 Hz anhand jeder der jeweiligen Phasen-Frequenz-Kurven;
Berechnen einer Differenz zwischen den Anstiegen $T_1$ und $T_2$, wobei die Differenz einer arteriellen Pulswellenlaufzeit für den Patienten zwischen dem proximalen und dem distalen Bereich entspricht; und
Speichern der arteriellen Pulswellenlaufzeit in dem Speicher.

11. System nach Anspruch 10, wobei das zeitlich variierende Quellen-Signal eine beliebige Kombination aus NIR-Bildern, RGB-Bildern, RGB- mit NIR-Bildern, multispektralen Bildern, Wärmebildern und hyperspektralen Bildern umfasst.

12. System nach Anspruch 10, wobei der proximale und der distale Bereich jeweils eine erste bzw. eine zweite lokal begrenzte Fläche des Patienten umfassen.

13. System nach Anspruch 10, das des Weiteren umfasst, dass anhand der arteriellen Pulswellenlaufzeit ein Blutdruck im Gefäßnetz des Patienten, eine Erweiterung der Blutgefäße im Verlauf der Zeit, eine Blutgefäß-Verstopfung oder/und das Vorhandensein einer peripheren Neuropathie bestimmt wird/werden.

14. System nach Anspruch 10, wobei das Video-Bildgebungssystem, das zum Erfassen der zeitlich variierenden Quellen-Bilder des proximalen und des distalen Bereiches eingesetzt wird, zwei Videokameras, das heißt eine erste Videokamera zum Erfassen eines ersten Videobildes des proximalen Bereiches und eine zweite Videokamera zum Erfassen eines zweiten Videobildes des distalen Bereiches umfasst.

**Revendications**

1. Procédé, mis en œuvre par un processeur, pour la détermination d'un temps de transit de pouls artériel entre une région proximale et une région distale d'un sujet d'intérêt à partir d'images vidéo source acquises à l'aide d'un système d'imagerie vidéo, le procédé comprenant :

la réception d'images sources variant dans le temps acquises sur au moins un canal d'un système d'imagerie vidéo, lesdites images sources comprenant des images vidéo capturées d'une région proximale et d'une région distale d'une zone de peau exposée d'un sujet d'intérêt, chacune des régions proximale et distale étant suffisamment grande pour que la contribution de capillaires et d'artérioles terminales améliore le rapport signal sur bruit, dans lequel la région proximale est le revers d'une main du sujet, et la région distale est le visage du sujet ;
l'extraction, à partir desdites images sources, d'un premier signal chronologique pour ladite région proximale et d'un second signal chronologique pour ladite région distale ;
le calcul d'un premier angle de phase $\varphi_1(\omega)$ en fonction de la fréquence $\omega$ dudit signal chronologique pour ladite région proximale pour obtenir une première courbe phase-fréquence ;
le calcul d'un second angle de phase $\varphi_2(\omega)$ en fonction de la fréquence $\omega$ dudit signal chronologique pour ladite région distale pour obtenir une seconde courbe phase-fréquence ;
le calcul de pentes respectives

$$T_1 = {}^{\partial\varphi_1}\!/_{\partial\omega} \text{ et } T_2 = {}^{\partial\varphi_2}\!/_{\partial\omega}$$

au sein d'une plage de fréquences cardiaques définies entre 0,75 et 4,0 Hz à partir de chacune desdites courbes phase-fréquence respectives ;
le calcul d'une différence entre lesdites pentes

$T_1$ et $T_2$, ladite différence correspondant à un temps de transit de pouls artériel pour ledit sujet entre lesdites régions proximale et distale ; et le stockage dudit temps de transit de pouls artériel dans un dispositif de stockage.

2. Procédé selon la revendication 1, dans lequel ledit signal source variant dans le temps comprend une quelconque combinaison parmi : des images en proche infrarouge, NIR, des images RVB, images RVB avec NIR, des images multispectrales, des images thermiques et des images hyperspectrales.

3. Procédé selon la revendication 1, dans lequel lesdites régions proximale et distale comprennent, respectivement, une première et une seconde zone localisée dudit sujet.

4. Procédé selon la revendication 1, comprenant en outre la détermination, à partir dudit temps de transit de pouls artériel, de l'une quelconque parmi : une pression sanguine dans le réseau vasculaire dudit sujet, une dilatation des vaisseaux sanguins au cours du temps, une obstruction des vaisseaux sanguins, une vitesse de circulation sanguine, et l'existence d'une neuropathie périphérique.

5. Procédé selon la revendication 1, dans lequel ledit système d'imagerie vidéo utilisé pour acquérir lesdites images sources variant dans le temps desdites régions proximale et distale comprend deux caméras vidéo, une première caméra vidéo acquérant une première vidéo de ladite région proximale et une seconde caméra vidéo acquérant une seconde vidéo de ladite région distale.

6. Procédé selon la revendication 1, dans lequel ladite source variant dans le temps est un canal de couleur unique.

7. Procédé selon la revendication 6, dans lequel ledit canal de couleur unique est un canal de couleur verte.

8. Procédé selon la revendication 1, dans lequel ledit calcul desdites pentes respectives inclut l'ajustement d'une équation polynomiale sur chacune desdites courbes phase-fréquence.

9. Procédé selon la revendication 1, dans lequel ledit calcul desdites pentes respectives inclut le calcul d'une dérivée par rapport à une fréquence.

10. Système basé sur des vidéos pour la détermination d'un temps de transit de pouls artériel entre une région proximale et une région distale d'un sujet d'intérêt à partir d'images vidéo source acquises à l'aide d'une caméra vidéo, le système comprenant :

un système d'imagerie vidéo pour la capture d'images sources variant dans le temps sur au moins un canal, lesdites images sources comprenant des images vidéo capturées d'une région proximale et d'une région distale d'une zone de peau exposée d'un sujet d'intérêt, chacune des régions proximale et distale étant suffisamment grande pour que la contribution de capillaires et d'artérioles terminales améliore le rapport signal sur bruit, dans lequel la région proximale est le revers d'une main du sujet, et la région distale est le visage du sujet ; et
un processeur en communication avec ladite caméra vidéo et une mémoire, ledit processeur étant configuré pour exécuter des instructions lisibles par machine pour la réalisation de :

la réception d'images sources variant dans le temps acquises sur au moins un canal dudit système d'imagerie vidéo, lesdites images sources comprenant des images vidéo capturées d'une région proximale et d'une région distale d'une zone de peau exposée d'un sujet d'intérêt ;
l'extraction, à partir desdites images sources, d'un premier signal chronologique pour ladite région proximale et d'un second signal chronologique pour ladite région distale ;
le calcul d'un premier angle de phase $\varphi_1(\omega)$ en fonction de la fréquence $\omega$ dudit signal chronologique pour ladite région proximale pour obtenir une première courbe phase-fréquence ;
le calcul d'un second angle de phase $\varphi_2(\omega)$ en fonction de la fréquence $\omega$ dudit signal chronologique pour ladite région distale pour obtenir une seconde courbe phase-fréquence ;
le calcul de pentes respectives

$$T_1 = \frac{\partial \varphi_1}{\partial \omega} \text{ et } T_2 = \frac{\partial \varphi_2}{\partial \omega} \text{ au}$$

sein d'une plage de fréquences cardiaques définies entre 0,75 et 4,0 Hz à partir de chacune desdites courbes phase-fréquence respectives ;
le calcul d'une différence entre lesdites pentes $T_1$ et $T_2$, ladite différence correspondant à un temps de transit de pouls artériel pour ledit sujet entre lesdites régions proximale et distale ; et
le stockage dudit temps de transit de pouls artériel dans ladite mémoire.

11. Système selon la revendication 10, dans lequel ledit signal source variant dans le temps comprend une quelconque combinaison parmi : des images en proche infrarouge, NIR, des images RVB, images RVB avec NIR, des images multispectrales, des images thermiques et des images hyperspectrales.

12. Système selon la revendication 10, dans lequel lesdites régions proximale et distale comprennent, respectivement, une première et une seconde zone localisée dudit sujet.

13. Système selon la revendication 10, comprenant en outre la détermination, à partir dudit temps de transit de pouls artériel, de l'une quelconque parmi : une pression sanguine dans le réseau vasculaire dudit sujet, une dilatation des vaisseaux sanguins au cours du temps, une obstruction des vaisseaux sanguins, une vitesse de circulation sanguine, et l'existence d'une neuropathie périphérique.

14. Système selon la revendication 10, dans lequel ledit système d'imagerie vidéo utilisé pour acquérir lesdites images sources variant dans le temps desdites régions proximale et distale comprend deux caméras vidéo, une première caméra vidéo pour l'acquisition d'une première vidéo de ladite région proximale et une seconde caméra vidéo pour l'acquisition d'une seconde vidéo de ladite région distale.

FIG. 1

START ⟋200

RECEIVE A TIME VARYING SOURCE VIDEO IMAGE OF A SUBJECT OF INTEREST ⟋202

PROCESS THE VIDEO IMAGES TO IDENTIFY A PROXIMAL AND DISTAL REGION OF THE SUBJECT WHICH CONTAIN EXPOSED SKIN ⟋204

EXTRACT VIDEO IMAGES ASSOCIATED WITH EACH OF THE PROXIMAL AND DISTAL REGIONS TO OBTAIN A TIME SERIES SIGNAL FOR EACH CHANNEL ⟋206

COMPUTE A PHASE ANGLE WITH RESPECT TO A FREQUENCY CURVE FOR EACH OF THE TIME SERIES SIGNALS TO OBTAIN PHASE CURVES ⟋208

EXTRACT, FROM EACH PHASE V/S FREQUENCY CURVE, RESPECTIVE SLOPES WITHIN A SELECTED CARDIAC FREQUENCY RANGE ⟋210

COMPUTE A DIFFERENCE BETWEEN THE SLOPES TO OBTAIN AN ARTERIAL PULSE TRANSIT TIME FOR THE SUBJECT ⟋212

STOP

FIG. 2

FIG. 3

FIG. 4

FIG. 5

EP 2 848 193 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130218028 A1
- US 247683, Mestha
- US 281975,  Mestha
- US 023310, Mestha
- US 967775, Wang
- US 086006, Wang
- US 324368, Wang
- US 401207, Mestha

**Non-patent literature cited in the description**

- **MESTHA.** *Removing Environment Factors From Signals Generated From Video Images Captured For Biomedical Measurements*